# EUROPÄISCHE PATENTSCHRIFT

(11) **EP 2 963 414 B1**
(45) Veröffentlichungstag und Bekanntmachung des Hinweises auf die Patenterteilung: **11.10.2017**
(21) Anmeldenummer: 15001947.9
(22) Anmeldetag: 30.06.2015
(51) Int. Cl.: G01N 27/64, G01N 33/18

(54) **FLÜSSIGKEITSSONDE**
FLUID PROBE
SONDE DE LIQUIDE

(30) Priorität: 30.06.2014 DE 102014109135
(43) Veröffentlichungstag der Anmeldung: 06.01.2016
(73) Patentinhaber: Leopold Siegrist GmbH, 76139 Karlsruhe (DE)
(72) Erfinder: Siegrist, Michael, 76139 Karlsruhe (DE)
(74) Vertreter: Geitz Truckenmüller Lucht Christ

(56) Entgegenhaltungen:
- WO-A1-2014/075724
- WO-A2-2006/102520
- DE-A1- 19 703 744
- DE-C2- 3 126 648
- DE-U1-202004 000 570
- OSER H ET AL: "Membrane introduction/laser photoionization time-of-flight mass spectrometry", CHEMOSPHERE, Bd. 67, Nr. 9, 12. März 2007 (2007-03-12), Seiten 1701-1708, XP005922170, ISSN: 0045-6535, DOI: 10.1016/J.CHEMOSPHERE.2006.05.117

## Beschreibung

Die Erfindung betrifft eine Flüssigkeitssonde zur Messung von leichtflüchtigen Stoffen in Flüssigkeiten mit einer gaspermeablen und hydrophoben Membran, die einen in die Flüssigkeitssonde integrierten Messraum von der zu analysierenden Flüssigkeit derart trennt, dass in der zu analysierenden Flüssigkeit gelöste Gase in Abhängigkeit von ihrer Löslichkeit und ihrem Dampfdruck durch diese Membran in den Messraum diffundieren, wobei dem Messraum (20) ein Photoionisationsdetektor (10) zugeordnet ist, der das in den Messraum (20) diffundierte Gas ionisiert und die resultierende Veränderung des Stromflusses zwischen zwei Elektroden in dem Messraum (20) erfasst.

Eine derartige Flüssigkeitssonde kann der DE 20 2004 000 570 U1 entnommen werden. Aus dieser Schrift ist bereits eine Anordnung zur Messung organischer Substanzen in Flüssigkeiten oder Dämpfen vorbekannt, bei der in einem wasserdichten Gehäuse in einem geführten Gasstrom ein Photoionisationsdetektor angeordnet ist und zur Realisierung verschiedener Betriebszustände mindestens ein Ventil und/oder zum Schutz vor Übersättigung mindestens ein Aktivkohlefilter angeordnet sind. Die vorbekannte Messsonde soll im Dauerbetrieb zur Messung leicht- und mittelflüchtiger organischer permeabler Substanzen in flüssigen Proben und Dampfphasen einsetzbar sein und dabei vor Schaden durch möglicherweise eintretendes Wasser geschützt sein.

Außerdem ist aus dem Fachaufsatz von Harald Oser et al. unter dem Titel "Membrane introduction/laser photoionization time-of-flight mass spectronomy", veröffentlicht in CHEMOSPHERE Band 67, Nr. 9, Seiten 1701-1708, ein Kombinationsgerät, umfassend eine Vorrichtung zur Multiphotonen-Ionisation in Verbindung mit einem Massenspektrometer zur Detektion organischer Anteile in Echtzeit zur Grund- und Trinkwasseranalyse vorbekannt.

Außerdem ist aus der DE 31 26 648 C2 eine Sonde zur unmittelbaren und kontinuierlichen Messung organischer Lösungsmittel in einer Flüssigkeit vorbekannt. Dabei arbeiten das vorbekannte Verfahren und die vorbekannte Vorrichtung mit einem sogenannten Halbleiter-Gassensor. Halbleiter-Gassensoren zur Gasdetektion bestehen üblicherweise aus Metalloxiden, wie etwa Zinkoxid, Zinnoxid oder Kupferoxid. Der Messeffekt beruht auf der Anlagerung von bestimmten Gasatomen an der Oberfläche des Halbleitermaterials, wodurch eine elektrische Widerstandsänderung hervorgerufen wird. Ein Problem dieser Sensoren besteht in deren Selektivität, da es schwierig ist, zu verhindern, dass sich auch die nicht zur Detektion vorgesehenen Gase an der Detektoroberfläche anlagern. Man versucht dieses Problem durch das Auftragen von Spezialbeschichtungen zu verhindern oder zumindest zu reduzieren, wobei im Weiteren der Halbleiter-Gassensor in einer Messzelle angeordnet ist, deren Messraum mittels einer flüssigkeitsundurchlässigen Membran von der die Messzelle umgebenden Flüssigkeit getrennt ist. Im Ergebnis ist also bei dem vorbekannten Verfahren wie auch bei der vorbekannten Vorrichtung vorgesehen, die Flüssigkeit in einem geschlossenen Messraum zu analysieren, also nicht etwa in einer fließenden Flüssigkeit.

Im Übrigen ist bei dem vorstehenden Verfahren systemimmanent die Detektionsgenauigkeit beschränkt und im Übrigen auf einige bestimmte zur Detektion geeignete Gase beschränkt.

Ausgehend von diesem Stand der Technik liegt der Erfindung die Aufgabe zu Grunde, eine verbesserte Flüssigkeitssonde zur Messung von leichtflüchtigen Stoffen in Flüssigkeiten zu schaffen, die sich insbesondere zur Analyse von durchfließenden Flüssigkeiten eignet und darüber hinaus eine höhere Genauigkeit besitzt, die beispielsweise auch die Analyse von Trinkwasser auf etwaige Verunreinigungen gestattet.

Die Lösung dieser Aufgabe gelingt dadurch, dass dem Messraum anstelle des Halbleiter-Gassensors ein Photoionisationsdetektor zugeordnet ist, der mit einem abweichenden Messprinzip dahingehend arbeitet, dass das durch die gas-permeable Membran in den Messraum diffundierte Gas ionisiert wird und die resultierenden Veränderungen eines Stromflusses zwischen zwei Elektroden in dem Messraum erfasst und an eine Auswerteelektronik übergeben werden, die ein korrespondierendes Ausgangssignal an eine Ausgangsschnittstelle der Flüssigkeitssonde übergeben. Der in diesem Zusammenhang erstmals eingesetzte Photoionisationsdetektor bietet eine deutlich höhere Genauigkeit, als die bisher in diesem Zusammenhang bekannt gewordenen Halbleiter-Gassensoren und erlaubt daher auch die Analyse durchfließender Flüssigkeiten im Hinblick auf die in diesen Flüssigkeiten gelösten Gase. Im Einzelnen besitzt der eingesetzte Photoionisationsdetektor eine Nachweisempfindlichkeit im ppb-Bereich. Der Messbereich des Photoionisationsdetektors liegt im Bereich von 0 - 50 ppb. Hierdurch können schon kleinste Verunreinigungen, etwa durch chlorierte Kohlenwasserstoffe (CKW), wie Trichlorethen, Tetrachlorethen, Dichlormethan u. a., detektiert werden. Dabei erfolgt die Ionisation des in den Messraum eindiffundierten Gases mit ausgewählten Beleuchtungselementen, wobei die Beleuchtungselemente von den zur Detektion vorgesehenen Gasen abhängig ausgewählt sind und somit sicherstellen, dass insbesondere die in die Flüssigkeit eingetragenen Gase mit der erforderlichen Sicherheit und Genauigkeit detektiert werden. So kann es sich beispielsweise bei den ausgewählten Beleuchtungselementen um UV-Lampen in einem speziellen Lichtspektrum handeln. Die UV-Lampe emittiert Ionen, die in den Messraum der Flüssigkeitssonde gelangen. Dabei werden diese Ionen von den in den eindiffundierten Gasen gelösten organischen und einigen anorganischen Substanzen absorbiert. Diejenigen Substanzen, die ein niedrigeres Ionisationspotential als die UV-Lichtquelle besitzen, werden hierbei ionisiert. Außerdem ist dem Messraum zusätzlich ein elektrochemischer Sensor zugeordnet. Hierdurch kann insbesondere eine zusätzliche Messung des Schwefelwasserstoffgehalts und/oder von des Anteils von schwefligen Gasen vorgenommen werden

In vorteilhafter Ausgestaltung ist die Flüssigkeitssonde mit einem, vorzugsweise zylinderförmigen, Edelstahlgehäuse versehen. Dies deshalb, weil es insbesondere im Bereich der Analyse von Flüssigkeiten, vornehmlich von Trinkwasser, eines korrosionsfesten Messgehäuses bedarf.

In konkreter Ausgestaltung ist die Flüssigkeitssonde eingangsseitig mit der bereits angesprochenen gaspermeablen, diffusionsoffenen Membran versehen, wobei die Membran den Querschnitt des Messraumes der Flüssigkeitssonde eingangsseitig überdeckt. In vorteilhafter Ausgestaltung ist dabei die Membran mit einem die Membran einfassenden O-Ring verklebt, wobei dieser O-Ring auf einer entsprechenden Einfassung der Einlauföffnung des Messraumes aufsetzbar ist, wobei dann diese Membran mittels einer, auf der dem Messraum abgewandten Seite aufgesetzten, ebenfalls diffusionsoffenen Haltemembran in dieser Einbausituation reversibel, etwa durch eine Verschraubung, gesichert ist. Durch die Einfassung der Membran mit einem O-Ring kann diese leicht gehandhabt und eingesetzt sowie bedarfsweise ausgewechselt werden. In diesem Zusammenhang ist es sinnvoll, dass die gaspermeable Membran ihrerseits mit einer Halteplatte gesichert ist, wobei die Halteplatte reversibel befestigt ist und beispielsweise nach Lösung der Verschraubung abgenommen werden kann, so dass dann auch die darunter angeordnete diffusionsoffene Membran, die die Einlauföffnung des Messraumes überdeckt, abgenommen und durch eine neue Membran ersetzt werden kann. Nachdem sich eine derartige Membran je nach Reinigungsgehalt der zu analysierenden Flüssigkeit vergleichsweise schnell zusetzt, muss diese in regelmäßigen Abständen gewechselt werden, so dass die hierdurch erleichterte Handhabbarkeit des Auswechselns der Membran sehr hilfreich ist.

In diesem Zusammenhang ist es sehr hilfreich, wenn die fragliche Membran als Teflonmembran ausgestaltet ist und somit über die erforderliche Stabilität verfügt. Darüber hinaus bietet die Teflonbeschichtung der Membran den Vorteil, dass sie in weitaus geringerem Ausmaß als vergleichbare Membranen etwaigen Anlagerungen ausgesetzt ist.

In weiterer Ausgestaltung ist die Flüssigkeitssonde über eine Ausgangsschnittstelle mit einer Anzeige- und Auswerteeinheit verbindbar, die in Abhängigkeit von dem erfassten Stromfluss bzw. dessen Änderungen die Konzentration der zu detektierenden Gase mit einer extrem hohen Genauigkeit anzeigt. In dem Messraum ist eine Elektrodenanordnung, umfassend eine positiv geladene Hochspannungselektrode und eine Kollektorelektrode, angeordnet. Die Hochspannungselektrode beschleunigt die ionisierten Gasmoleküle in Richtung der Kollektorelektrode. Der resultierende Ionenstrom steht in direkter Abhängigkeit von der jeweiligen Konzentration der in dem eindiffundierten Gasstrom gelösten, zu detektierenden Substanzen und wird im Weiteren detektiert und in der Auswerteeinheit erfasst und ausgewertet.

In vorteilhafter Weiterbildung kann der Messraum der Flüssigkeitssonde als ein geschützter separater Raum ausgebildet sein. In diesem Falle werden die durch die Membran eindiffundierten Gase mittels einer Mikropumpe in diesen geschützten Messraum gepumpt. Diese Lösung kann in solchen Fällen sinnvoll sein, in denen die Gefahr besteht, dass durch Kondensation, etwa infolge des Temperaturgefälles zwischen der zu überwachenden Flüssigkeit und der Umgebungstemperatur, insbesondere der Temperatur im Messraum, die Gefahr besteht, dass Flüssigkeit innerhalb des Messraumes kondensiert und die Messung verfälscht und/oder die Elektronik beschädigt. Im Rahmen einer alternativen Lösung könnte auch der Messkopf der Flüssigkeitssonde beheizt werden.

In vorteilhafter Weiterbildung dieser Lösung kann in definierten zeitlichen Abständen in den geschützten Messraum Umgebungsluft gepumpt werden, um auf diesem Weg eine Nullreferenz für die mit der Flüssigkeitssonde vorzunehmenden Messungen zu gewinnen.

In abermals vorteilhafter Weiterbildung dieser Lösung kann die Nullreferenz nicht nur zur Erhöhung der Messgenauigkeit eingesetzt werden, sondern auch dazu benutzt werden, die unvermeidbare Drift der innerhalb des Photoionisationsdetektors eingesetzten UV-Lampen im Wege einer Nachkalibrierung zu berücksichtigen, um auf diesem Weg über einen längeren Zeitraum die erforderliche Messgenauigkeit aufrecht zu erhalten. Hierdurch können auch die Einsatzzeiten der innerhalb des Photoionisationsdetektors eingesetzten UV-Lampen verlängert werden, bzw. die Lebensdauer der Flüssigkeitssonde verlängert werden.

In einer sinnvollen Ausgestaltung kann in die in die Flüssigkeitssonde integrierte Auswerteelektronik auch ein Schwellwertgeber eingearbeitet sein, der im Rahmen der Messungen, etwa im Rahmen einer Überwachung, lediglich auf die Überschreitung voreingestellter Grenzwerte anschlägt und über die Ausgangsschnittstelle an eine Anzeigeeinheit übermittelt.

Im Übrigen kann es sinnvoll sein, den Photoionisationsdetektor und/oder elektrochemischen Sensor jeweils drahtlos mit der Auswertelektronik zu verbinden. Außerdem können in diesem mehrere Messsonden zu einem Sondennetzwerk zusammengefasst werden. Dies wäre ein wesentlicher Beitrag zur Erhöhung der Messsicherheit und -genauigkeit.

Ein weiterer Vorteil des Photoionisationsdetektors, etwa im Vergleich zu einem Halbleitergasdetektor, ist seine schnelle Reaktionszeit kleiner 10 sec. und sein geringer Strombedarf. Letzteres erlaubt es, die Flüssigkeitssonde als mobile Einheit zu nutzen, indem ein Stromspeicher zusätzlich in die Sonde zur Bereitstellung der Betriebsspannung austausch- und/oder aufladbar integriert wird.

Aufgrund der hohen Genauigkeit der vorstehend beschriebenen Flüssigkeitssonde kann diese auch zur kontinuierlichen Überwachung fließender Medien auf in diesem Medium aufgelöste gasförmige Inhaltsstoffe, vorzugsweise auf chlorierte Kohlenwasserstoffe und/oder Aromate, eingesetzt werden.

In konkreter Anwendung kann die Flüssigkeitssonde in eine zu überwachende Trinkwasserleitung zur Überwachung der Trinkwasserversorgung insgesamt bzw. einer abgeschlossenen Einheit derart eingesetzt werden, dass die Flüssigkeitssonde in der Zulaufleitung oder in den Zulaufkanal derart platziert wird, dass die mit der gaspermeablen und hydrophoben Membran verschlossene Eingangsseite der Flüssigkeitssonde der Fließrichtung des Trinkwassers entgegengewandt ist. Mit einer solchen Anordnung kann eine kontinuierliche Überwachung des Trinkwassers auf die Überschreitung vorgegebener Grenzwerte durchgeführt werden, wobei lediglich darauf zu achten ist, dass in regelmäßigen Abständen die diffusionsoffene Membran ausgetauscht wird, wobei die ebenfalls vorstehend beschriebene leichte Auswechselbarkeit dieser Membran sowie deren verbesserte Haptik durch die Verklebung mit einem O-Ring den entsprechenden Membranwechsel spürbar erleichtert.

Die Erfindung wird anhand eines in der Zeichnung nur schematisch dargestellten Ausführungsbeispiels näher erläutert.

Es zeigen:
- Figur 1: eine Flüssigkeitssonde in einer Querschnittansicht und
- Figur 2: die Flüssigkeitssonde gemäß Figur 1 im Messeinsatz in einer Prinzipskizze.

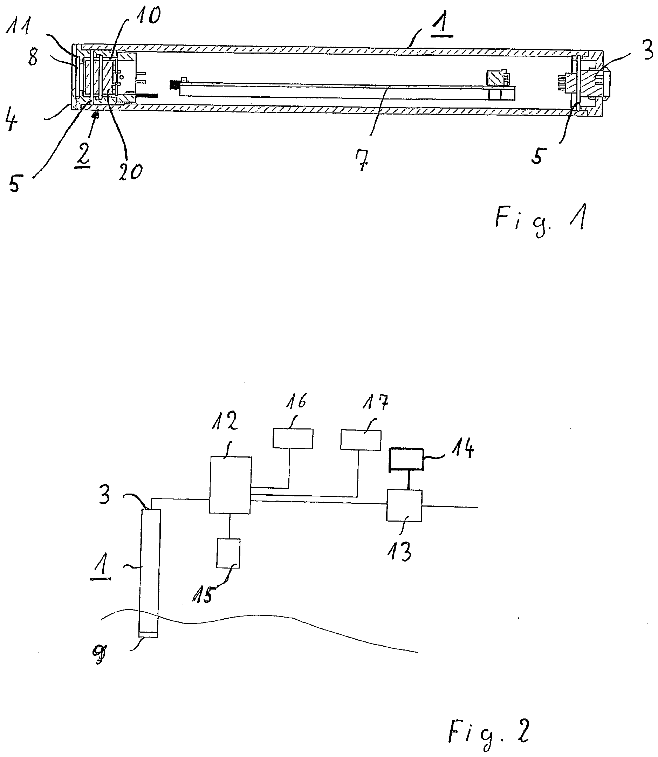

Die in Figur 1 gezeigte Flüssigkeitssonde besteht zunächst aus einem zylindrischen Rohr 1 aus korrosionsfestem Edelstahl, in das eingangsseitig ein Sensoroberteil 2 mit einem integrierten Photoionisationsdetektor 10 eingesetzt werden kann. Dabei ist der eigentliche Messraum 20 der Flüssigkeitssonde mit einer gaspermeablen und hydrophoben Teflonmembran 8 verschließbar, wobei diese Teflonmembran 8 auf einen O-Ring 11 geklebt ist und in eine entsprechende Einfassung der Abdeckung des Messraums 20 eingesetzt und mittels geeigneter Zylinderschrauben in dieser Position gesichert werden kann. Darüber hinaus wird auf diese Teflonmembran 8 auf der von dem Photoionisationsdetektor 10 abgewandten Seite eine Halteplatte 4 aufgesetzt, die ihrerseits mittels Kreuzschlitzschrauben gesichert ist.

Dieses Sensoroberteil 2 kann dann insgesamt flüssigkeitsdicht in das Zylinderrohr 1 eingesetzt werden, wobei dabei das Zylinderrohr 1 selbst mittels einer weiteren Abdichtung 5 flüssigkeitsdicht abgeschlossen ist. Im Übrigen ist in das Zylinderrohr 1 eine Auswerteelektronik in Form der Platine 7 integriert.

Auf der von der mit der Teflonmembran 8 verschlossenen Eingangsseite des Flüssigkeitssensors abgewandten Stirnseite ist das Zylinderrohr 1 mit einem Abschlussstecker 3 versehen, der die Ausgangsschnittstelle des Flüssigkeitssensors darstellt. Auch dieser Abschlussstecker 3 wird auf der von der Eingangsseite 9 abgewandten Seite des Zylinderrohrs 1 eingesetzt und flüssigkeitsdicht mit dem Zylinderrohr 1 verschraubt. Der Abschlussstecker 3 ist seinerseits flüssigkeitsfest mit einem Einsteckeinsatz, der mit einer Zylinderkopfschraube mit dem Abschlussstecker 3 verschraubt wird, verschlossen.

Nach einer entsprechenden Kalibrierung der Flüssigkeitssonde kann diese dann gemäß der Darstellung in Figur 2 derart in eine strömende Flüssigkeit eingesetzt werden, dass die Eingangsseite 9 des Zylinderrohrs 1 entgegen der Strömungsrichtung ausgerichtet ist, so dass der in das Zylinderrohr 1 integrierte Messraum 20 ständig von Flüssigkeit durchströmt ist. Dabei dringen die in der Flüssigkeit gelösten Gase in Abhängigkeit von ihrem Gasdruck durch die permeable Teflonmembran 8 und gelangen damit in den Einflussbereich des Photoionisationsdetektors 10, der dem Messraum 20 zugeordnet ist. Der Photoionisationsdetektor 10 umfasst auf die jeweils zu detektierenden Gase ausgelegte Beleuchtungselemente, beispielsweise UV-Lampen, die in einem gewissen Lichtspektrum ausstrahlen und somit für eine Ionisation der einströmenden Gase sorgen, wodurch sich zwischen den zwei Elektroden des Photoionisationsdetektors 10 eine Änderung des Stromflusses ausbildet, die erfasst und in ein korrespondierendes Signal mit der auf der Platine 7 angeordneten Auswerteelektronik umgesetzt wird, das an der Ausgangsschnittstelle, die durch den Abschlussstecker 3 repräsentiert ist, abgeleitet werden kann und im Weiteren über eine Verteilereinheit 12 über einen Schwellwertgeber 13 an eine Anzeigeeinheit 14 oder eine sonstige Signaleinheit übermittelt werden kann. Auf diesem Weg kann die etwaige Überschreitung definierter Grenzwerte angezeigt werden.

Dabei ist der Abschlussstecker 3 über die Verteilereinheit 12 an eine Stromversorgung 15 angeschlossen.

Zusätzlich sind über die Verteilereinheit 12 eine analoge und eine serielle Schnittstelle 16 und 17 an den Abschlussstecker 3 angeschlossen. Durch diese Schnittstellen 16, 17 können zusätzlich oder alternativ zur Anzeige von Grenzwertüberschreitungen über den Schwellwertgeber 13 auch Konzentrationen der in der zu analysierenden Flüssigkeit enthalten gelösten Gase, also etwa von chlorierten Kohlenwasserstoffen und/oder Aromaten, erfolgen.

Vorstehend ist somit ein robuster und einfach zu handhabender Flüssigkeitssensor beschrieben, der sich zur ständigen Überwachung von Trinkwasserleitungen, insbesondere auf die Überschreitung von vorgegebenen Grenzwerten, eignet, aber auch zu einer ständigen Überwachung der Trinkwasserqualität, etwa durch eine ständige Ermittlung und Aufzeichnung der zur Detektion vorgesehenen, in der zu überwachenden Flüssigkeit gelösten Gase.

## Patentansprüche

1. Flüssigkeitssonde zur Messung von leichtflüchtigen Stoffen in Flüssigkeiten mit einer gaspermeablen und hydrophoben Membran, die einen in die Flüssigkeitssonde integrierten Messraum (20) von der zur analysierenden Flüssigkeit derart trennt, dass in der zu analysierenden Flüssigkeit gelöste Gase in Abhängigkeit von ihrer Löslichkeit und ihrem Dampfdruck durch diese Membran in den Messraum (20) diffundieren, wobei dem Messraum (20) ein Photoionisationsdetektor (10) zugeordnet ist, der das in den Messraum (20) diffundierte Gas ionisiert und die resultierende Veränderung des Stromflusses zwischen zwei Elektroden in dem Messraum (20) erfasst,
**dadurch gekennzeichnet, dass**
der Photoionisationsdetektor (10) diese resultierende Veränderung des Stromflusses an eine Auswerteelektronik übergibt, die ein korrespondierendes Ausgangssignal an eine Ausgangsschnittstelle der Flüssigkeitssonde übergibt, wobei der Photoionisationsdetektor (10) zur Ionisation der in den Messraum (20) eindiffundierten Gase mit in Abhängigkeit von den zu analysierenden gelösten Gasen ausgewählten Beleuchtungselementen, etwa UV-Lampen, bestückt ist, wobei dem Messraum (20) zusätzlich ein elektrochemischer Sensor zugeordnet ist.

2. Flüssigkeitssonde nach Anspruch 1, **dadurch gekennzeichnet, dass** die Flüssigkeitssonde ein, vorzugsweise zylinderförmiges, Edelstahlgehäuse aufweist.

3. Flüssigkeitssonde nach Anspruch 1 oder 2, **dadurch gekennzeichnet, dass** die Flüssigkeitssonde eingangsseitig mit einer diffusionsoffenen Membran derart versehen ist, dass diese Membran den Querschnitt des Messraumes (20) der Flüssigkeitssonde eingangsseitig überdeckt und mit einem diese Membran einfassenden O-Ring (11) verklebt ist und dieser O-Ring (11) auf einer entsprechenden Einfassung des Messraumes (20) aufsetzbar ist und diese Membran mittels einer auf der dem Messraum (20) abgewandten Seite auf diese Membran aufgesetzten diffusionsoffenen Halteplatte (4) in dieser Einbausituation reversibel gesichert ist.

4. Flüssigkeitssonde nach Anspruch 3, **dadurch gekennzeichnet, dass** die gaspermeable und hydrophobe Membran als Teflonmembran (8) ausgestaltet ist.

5. Flüssigkeitssonde nach einem oder mehreren der vorhergehenden Ansprüche, **dadurch gekennzeichnet, dass** der Messraum (20) als geschützter separater Raum ausgebildet ist, wobei die eindiffundierten Gase mittels einer Mikropumpe in den geschützten Messraum gepumpt werden.

6. Flüssigkeitssonde nach Anspruch 5, **dadurch gekennzeichnet, dass** zur Ausbildung einer Nullreferenz in definierten zeitlichen Abständen, gereinigte Umgebungsluft in den geschützten Messraum gepumpt wird.

7. Flüssigkeitssonde nach Anspruch 6, **dadurch gekennzeichnet, dass** mittels der Nullreferenz in definierten zeitlichen Abständen eine Nachkalibrierung des Photoionisationsdetektors (10) ausführbar ist.

8. Flüssigkeitssonde nach einem oder mehreren der vorhergehenden Ansprüche, **dadurch gekennzeichnet, dass** die Flüssigkeitssonde über eine Ausgangsschnittstelle mit einer Anzeige- und Auswerteeinheit (14) verbindbar ist.

9. Flüssigkeitssonde nach einem oder mehreren der vorhergehenden Ansprüche, **dadurch gekennzeichnet, dass** die in die Flüssigkeitssonde integrierte Auswerteelektronik einen Schwellwertgeber (13) aufweist und die Flüssigkeitssonde dementsprechend über die Ausgangsschnittstelle mit einer Anzeigeeinheit (14) verbindbar ist.

10. Flüssigkeitssonde nach einem oder mehreren der vorhergehenden Ansprüche, **dadurch gekennzeichnet, dass** der Photoionisationsdetektor (10) und/oder der elektrochemische Sensor kabellos ausgebildet sind und ihre Messergebnisse drahtlos an die Auswerteelektronik übermittelt

11. Verwendung der Flüssigkeitssonde nach einem oder mehreren der vorhergehenden Ansprüche 1 bis 10 in einem fließenden Medium zur kontinuierlichen Überwachung dieses Mediums aufgelöste gasförmige Inhaltsstoffe, vorzugsweise chlorierte Kohlenwasserstoffe und/oder Aromate.

12. Verwendung der Flüssigkeitssonde nach Anspruch 11 zur Überwachung der Trinkwasserversorgung, insbesondere durch Anordnung der Flüssigkeitssonde in einer Trinkwasserleitung derart, dass die mit der gaspermeablen und hydrophoben Membran versehene Eingangsseite (9) der Flüssigkeitssonde der Fließrichtung des Trinkwassers entgegen gewandt ist.

## Claims

1. A liquid probe for measuring volatile materials in liquids, having a gas-permeable and hydrophobic membrane, which separates a measuring chamber (20) integrated into the liquid probe from the liquid to be analyzed such that gases dissolved in the liquid to be analyzed diffuse through this membrane into the measuring chamber (20) as a function of the solubility thereof and the vapor pressure thereof, wherein a photoionization detector (10) is associated with the measuring chamber (20), which ionizes the gas diffused into the measuring chamber (20) and detects the resulting change of the current flow between two electrodes in the measuring chamber (20),
**characterized in that** the photoionization detector (10) transmits this resulting change of the current flow to an analysis electronics system, which transmits a corresponding output signal at an output interface of the liquid probe, wherein the photoionization detector (10) for ionization of the gases diffused into the measuring chamber (20) is equipped with illumination elements, such as UV lamps, selected in accordance with the dissolved gases to be analyzed, wherein an electrochemical sensor is additionally associated with the measuring chamber (20).

2. The liquid probe according to Claim 1, **characterized in that** the liquid probe has a preferably cylindrical stainless-steel housing.

3. The liquid probe according to Claim 1 or 2, **characterized in that** the liquid probe is provided on the inlet side with a diffusion-open membrane such that this membrane covers the cross section of the measuring chamber (20) of the liquid probe on the inlet side and is adhesively bonded to an O-ring (11) bordering this membrane and this O-ring (11) can be placed on a corresponding border of the measuring chamber (20) and this membrane is reversibly secured in this installation situation by means of a diffusion-open holding plate (4) placed on this membrane on the side facing away from the measuring chamber (20).

4. The liquid probe according to Claim 3, **characterized in that** the gas-permeable and hydrophobic membrane is embodied as a Teflon membrane.

5. The liquid probe according to one or more of the preceding claims, **characterized in that** the measuring chamber (20) is designed as a protected separate chamber, wherein the gases diffused in are pumped by means of a micropump into the protected measuring chamber.

6. The liquid probe according to Claim 5, **characterized in that** filtered ambient air is pumped into the protected measuring chamber to form a zero reference at defined time intervals.

7. The liquid probe according to Claim 6, **characterized in that** a recalibration of the photoionization detector (10) is executable by means of the zero reference at defined time intervals.

8. The liquid probe according to one or more of the preceding claims, **characterized in that** the liquid probe is connectable via an output interface to a display and analysis unit (14).

9. The liquid probe according to one or more of the preceding claims, **characterized in that** the analysis electronics system integrated into the liquid probe has a threshold value encoder (13) and the liquid probe is accordingly connectable via the output interface to a display unit (14).

10. The liquid probe according to one or more of the preceding claims, **characterized in that** the photoionization detector (10) and/or the electrochemical sensor are designed as wireless and transmit the measurement results thereof wirelessly to the analysis electronics unit.

11. A use of the liquid probe according to one or more of preceding Claims 1 to 10 in a flowing medium for continuously monitoring gaseous contents dissolved in this medium, preferably chlorinated hydrocarbons and/or aromatics.

12. The use of the liquid probe according to Claim 11 for monitoring the drinking water supply, in particular by arranging the liquid probe in a drinking water line such that the inlet side (9) of the liquid probe provided with the gas-permeable and hydrophobic membrane is facing toward the flowing direction of the drinking water.

## Revendications

1. Sonde de liquide pour la mesure de substances très volatiles dans des liquides avec une membrane perméable aux gaz et hydrophobe qui sépare un espace de mesure (20) intégré dans la sonde de liquide du liquide à analyser de telle manière que des gaz dissous dans le liquide à analyser diffusent dans l'espace de mesure (20) à travers la membrane en fonction de leur solubilité et de leur pression de vapeur, l'espace de mesure (20) étant associé à un détecteur de photoionisation (10) qui ionise le gaz diffusé dans l'espace de mesure (20) et détecte le changement en résultant du courant entre deux électrodes dans l'espace de mesure (20),
**caractérisée en ce que** le détecteur de photoionisation (10) transmet ce changement du courant résultant à un circuit électronique d'analyse qui transmet un signal de sortie correspondant à une interface de sortie de la sonde de liquide, le détecteur de photoionisation (10) étant équipé, pour l'ionisation des gaz diffusés dans l'espace de mesure (20), d'éléments d'éclairage choisis en fonction des gaz dissous à analyser, par exemple de lampes à UV, un capteur électrochimique étant en outre associé à l'espace de mesure (20).

2. Sonde de liquide selon la revendication 1, **caractérisée en ce qu'**elle présente un boîtier en acier inoxydable, de préférence cylindrique.

3. Sonde de liquide selon la revendication 1 ou 2, **caractérisée en ce qu'**elle est munie du côté de l'entrée d'une membrane ouverte à la diffusion, de telle façon que cette membrane recoure la section de l'espace de mesure (20) de la sonde de liquide du côté de l'entrée et est collée à un joint torique (11) encadrant cette membrane et ce joint torique (11) peut être posé sur une monture correspondante de l'espace de mesure (20) et cette membrane est fixée de façon réversible dans cette situation de montage au moyen d'une plaque de maintien (4) ouverte à la diffusion posée sur cette membrane du côté opposé à l'espace de mesure (20).

4. Sonde de liquide selon la revendication 3, **caractérisée en ce que** la membrane perméable aux gaz et hydrophobe est réalisée comme une membrane en Téflon (8).

5. Sonde de liquide selon une ou plusieurs des revendications précédentes, **caractérisée en ce que** l'espace de mesure (20) est réalisé comme un espace protégé séparé, les gaz diffusés étant injectés dans l'espace de mesure protégé au moyen d'une micropompe.

6. Sonde de liquide selon la revendication 5, **caractérisée en ce qu'**afin de former une référence à zéro, de l'air ambiant purifié est injecté dans l'espace de mesure protégé à des intervalles de temps définis.

7. Sonde de liquide selon la revendication 6, **caractérisée en ce qu'**un réétalonnage du détecteur de photoionisation (10) peut être réalisé au moyen de la référence à zéro à des intervalles de temps définis.

8. Sonde de liquide selon une ou plusieurs des revendications précédentes, **caractérisée en ce que** la sonde de liquide peut être raccordée par une interface de sortie à une unité d'affichage et d'analyse (14).

9. Sonde de liquide selon une ou plusieurs des revendications précédentes, **caractérisée en ce que** le circuit électronique d'analyse intégré dans la sonde de liquide comporte un générateur de valeur de seuil (13) et la sonde de liquide peut être connectée en conséquence à une unité d'affichage (14) par l'interface de sortie.

10. Sonde de liquide selon une ou plusieurs des revendications précédentes, **caractérisée en ce que** le détecteur de photoionisation (10) et/ou le capteur électrochimique sont réalisés sans fil et leurs résultats de mesure sont transmis sans fil au circuit électronique d'analyse.

11. Utilisation de la sonde de liquide selon une ou plusieurs des revendications 1 à 10 dans un fluide en circulation pour la surveillance continue de composants gazeux dissous dans ce fluide, de préférence d'hydrocarbures chlorés et/ou aromatiques.

12. Utilisation de la sonde de liquide selon la revendication 11 pour la surveillance de l'adduction d'eau potable, en particulier par disposition de la sonde de liquide dans une conduite d'eau potable de sorte que le côté d'entrée (9) de la sonde de liquide munie de la membrane perméable aux gaz et hydrophobe soit tourné dans le sens opposé au sens d'écoulement de l'eau potable.
